# EUROPEAN PATENT SPECIFICATION

(11) **EP 3 265 139 B1**
(45) Date of publication and mention of the grant of the patent: **01.06.2022**
(21) Application number: 16707800.5
(22) Date of filing: 03.03.2016
(51) Int. Cl.: A61L 15/32, A61L 26/00, C08J 3/24, C08G 69/48, C12N 11/02, C12N 11/08

(54) **MICROPARTICLES**
MIKROPARTIKEL
MICROPARTICULES

(30) Priority: 03.03.2015 GB 201503566
(43) Date of publication of application: 10.01.2018
(73) Proprietor: Spheritech Ltd, Runcorn, Cheshire WA7 4QX (GB)
(72) Inventor: WELLINGS, Donald A, Cheshire WA7 4QX (GB)
(74) Representative: Geary, Stephen
(86) International application number: PCT/EP2016/054603
(87) International publication number: WO 2016/139322

(56) References cited:
- WO-A1-2012/143508
- DE-A1-102006 016 307
- DATABASE WPI Week 201014 Thomson Scientific, London, GB; AN 2010-B75370 XP002758241, & JP 2010 031197 A (TORAY IND INC) 12 February 2010 (2010-02-12)

## Description

This invention relates to microparticles, in particular to self-assembled microparticles, a method of preparing the microparticles, preparation of macroporous materials formed by collision of said particles and the use of the particles and porous structures obtained. The microparticles and porous materials are useful in a wide range of physical and chemical processes especially where interaction with a substrate is required for example solid phase synthesis, solid phase extraction, solid phase reagents, immobilisation of species, for example proteins and nucleic acids, cell culture, wound care including internal and external chronic wounds and acute wounds, treatment of burns, medical diagnostics, regenerative medicine, sight correction, controlled release of chemicals, for example drugs and agrochemicals, catalysis and chromatography.

Solid support materials useful in solid phase synthetic processes are known. A wide range of physical and chemical processes employ solid support materials including by way of example synthesis of organic molecules, in particular peptides and oligonucleotides, immobilisation of species, support of catalysts, ion exchange, extraction of species from a material, diagnostics and chromatography.

Typically, multi-stage synthesis of an organic molecule involves numerous isolation steps to separate intermediates, produced at each stage, before progressing to the subsequent stage. These processes are often time-consuming, expensive and may be inefficient as regards yield. The intermediates often require purification to remove excess reagents and reaction by-products and procedures such as precipitation, filtration, biphase solvent extraction; solid phase extraction, crystallization and chromatography may be employed.

Solid phase synthesis offers some advantages over solution phase synthesis. For example, isolation procedures used in solution phase synthesis may to some extent be avoided by reversibly attaching the target molecule to a solid support. Excess reagents and some of the side-products may be removed by filtration and washing of the solid support. The target molecule may be recovered in essentially quantitative yield in some processes which is typically particularly difficult in solution phase synthesis. In addition, the time required to perform operations on a solid support is typically much less than that required carrying out the equivalent stage in a solution phase synthesis.

Immobilisation of species in a range of processes is also known. For example, polymer supports are commonly used for the immobilisation of catalysts for use in traditional organic chemistry including chemo and bio catalysis. Immobilised enzymes may be employed to perform organic chemical reactions or for chiral resolution, for example the use of immobilised Penicillin amidase for the resolution of secondary alcohols (E. Baldaro et al. Tet. Asym. 4, 1031, (1993) and immobilised Penicillin G amidase is also used for the hydrolysis of Benzylpenicillin in the manufacture of Amoxicillin (Carleysmith, S. W. and Lilly, M.D.. Biotechnol. Bioeng., 21, 1057-73, 1979).

Solid supports are also used to immobilise biological macromolecules for medical and diagnostic applications. This includes immobilisation of proteins, monoclonal and polyclonal antibodies. Cell culture is commonly carried out on solid supports with specific surface characteristics and morphology. Immobilised enzymes can be employed as sensors to generate a signal. An example is the detection of glucose by the glucose oxidase/peroxidase coupled enzyme system, in which the presence of glucose generates hydrogen peroxide which in turn is the substrate for peroxidase for the oxidation of a wide variety of substrates to provide a coloured, fluorescent or luminescent signal.

A variety of fluors whose fluorescence is sensitive to specific cations or anions may be utilised to indicate concentrations of specific ions including hydrogen ions for pH measurement.

Polymeric particles and porous materials are often used in chromatography where the solid supports are termed stationary phases. In certain modes of chromatography the cost of stationary phases may be restrictive. In other modes the physical nature of the stationary phase can reduce the effectiveness of the technology. For instance, the soft polymers often used for affinity, ion-exchange and gel permeation chromatography cannot be used at high flow rates because of the deformable nature of the particles. The rigid macroporous polymers used for many other modes of chromatography can often be mechanically friable and subsequently suffer from a short lifetime.

The application of solid supports or stationary phases in chromatographic separations is very extensive for example complex high-technology separations used in the pharmaceutical and biotechnology industry and larger scale processes used in the mining industry. Some of the pharmaceutical industry's most valuable drugs are purified by preparative chromatography and improved chromatographic separation would be technically beneficial and economically advantageous. In the mining and precious metal recovery industry a large portion of the world's palladium, a critical component in a wide range of industrial applications and processes including catalytic converters and manufacture of high value products, may be refined using immobilised crown ethers (Traczyk, F.P.; Bruening, R.L.; Izatt, N.E. "The Application of Molecular Recognition Technology (MRT) for Removal and Recovery of Metal Ions from Aqueous Solutions"; In Fortschritte in der Hydrometallurgie; 1998, Vortrage beim 34. Metallurgischen Seminar des Fachausschusses fuer Metallurgische Aus-und Weiterbildung der GDMB; 18-20 November 1998; Goslar).

The use of polymeric particles and macroporous materials in solid phase extraction and in the preparation of solid phase reagents is also known in the chemical, pharmaceutical and biotechnology industry.

Known solid phase supports generally comprise polymer particles of a particular size and physical nature to suit the application. For ease of use these polymer particles are often spherical and have a defined particle size distribution. The spherical nature of the particles improves the flow and filtration characteristics of the polymer. Although the uses of solid supports have operational advantages there are disadvantages to the solid phase approach. For example, commercially available supports commonly used for solid phase synthesis of peptides and oligonucleotides may be expensive, for example due to the complex manufacturing processes. Microporous polymer particles and macroporous polymers are generally used. Microporous polymers have a relatively low level of cross-linker which allows the polymer particles to solvate and consequently swell in suitable solvents. Macroporous polymers have a high level of cross-linker in the polymer matrix and contain large pores. These polymer particles are generally rigid and have good flow characteristics and are suitable for use in packed columns.

Treatment of wounds and wound care present a major clinical challenge. Many known wound care treatments employ animal-derived collagen, for example co, horse, pig and human-derived collagen. The use of these materials may present a range of challenges based on ethical, moral and religious grounds and which hinders their widespread use. Animal-derived collagens may also present technical and commercial problems as extraction and processing of these products may be complex, slow and expensive production. Where a particular source of an animal-derived product is required, for example certain commercially available products rely on the use of horse tendons, availability of supply may present difficulties. Manufacture of animal-derived products may generate biological waste and require particular handling to reduce the risk of biological contamination.

A need exists for materials suitable for use in wound care applications which do not contain components derived from animals yet which are biocompatible, biodegradable, non-cytotoxic and desirably antimicrobial.

Known polymeric particles which are employed in many of these fields of use have certain drawbacks. Polymeric particles may typically be made by a dispersion or emulsion polymerisation process in which a solution of monomers is dispersed in an immiscible solvent (continuous phase) prior to initiation of the polymerisation. The polymer particles formed are typically then filtered, washed and classified to isolate the required particle size distribution. The process may however be complex and costly and be limited by the need to use organic solvents. The term "polymer" as employed herein includes inorganic polymers, for example silica and organic polymers, for example polyamide.

These processes are disadvantageous in some respects including monomer loss to the continuous phase, generation of a range of particle sizes and the undesirable generation of fine particles during the polymerisation leads to laborious particle size classification, for example by sieving or air classification.

In addition to undesirable costs of manufacture and wastage during preparation certain disadvantages may arise with the physical properties of the known polymeric particles. Microporous polymeric particles are generally soft and generally not suitable for use in chromatography applications at a high flow rate in a packed column bed. In addition, the soft particles may be compressed undesirably and cause fouling, for example during filtration often leading to compressive intrusion into the sinter or mesh being used at the bottom of the column. Rigid macroporous and macroreticular particles are more suited to high flow rates in packed column beds. However, due to the rigid nature the particles may be fragile and fragment under physical stress.

These problems are exacerbated by packing the polymer particles into the column from the bottom upwards such that the polymer particles are subjected to undesirably large stresses.

Spherulites typically comprise multiple layers of surfactants as an oil in water composition and may require the use of solvents and complex temperature control to form spherulites. The particle size may extend over a wide range.

We have now found that these and other problems associated with known polymer particles, macroporous materials and known spherulites may be ameliorated by providing a particulate support comprising a self-assembled microparticle comprising a fatty acid having two or more carboxylic acid groups and a base which provide a narrow particle size distribution or macroporous material formed by contacting self-assembled microparticles.

In a first aspect, the invention provides a self-assembled microparticle, a use and a method as defined in the accompanying claims. The microparticle comprises an acid of general formula HOOC-(CH₂)ₙ-COOH wherein n is at least 5 and not more than 40 and an organic base in which the molar ratio of acid groups to basic groups in the acid and base is from 0.6 to 1.4:1, the microparticle being obtainable by a process comprising contacting the said acid and organic base in aqueous solution, wherein the acid is insoluble in the aqueous solution and the organic base is soluble in the aqueous solution wherein the microparticle comprises a multi-lamellar structure.

The solvent is an aqueous solution.

Advantageously, an aqueous-based solvent, preferably water, allows the microparticle to be used in applications in which environmental considerations are important. For example, the microparticle may be formulated into an aqueous-based product which may be suitable for personal use or consumption, medical uses and for example as a biocide. Known biocides may contain environmentally undesirable solvents or components, for example isopropanol and silicone which requires careful use and disposal and subsequent cleaning for example when used in cleaning or antimicrobial applications. A water-based composition as provided for by this invention reduces or avoids disadvantages associated with products containing organic solvent or silicone.

The bis-aliphatic acid comprises a bis-carboxylic fatty acid in which terminal carboxylic acids are linked by a region which is less hydrophilic than the terminal carboxylic acids and is preferably hydrophobic. The less hydrophilic region may comprise a backbone with substituents and/or the backbone may comprise heteroatoms, for example poly-epsilon lysine.

The region linking the carboxylic acids is hydrophobic and is a hydrocarbyl group. The hydrophobic group is an aliphatic hydrocarbyl group. The bis-acid is a compound of general formula HOOC-(CH₂)ₙ-COOH which is insoluble in water and n is preferably at least 6, especially at least 7. Suitably n is not more than 36, more preferably not more than 25, and especially not more than 20. Preferably n is from 7 to 18.

In a preferred embodiment, the organic acid comprises a C₇ to C₁₈ bis carboxylic fatty acid. In another preferred embodiment, the organic acid comprises a C₇ to C₁₃ bis carboxylic fatty acid together with a further acid selected from a EDTA, nitrolotriacetic acid and a monocarboxylic acid, preferably a C₆ to C₁₈ carboxylic acid, for example caproic acid, palmitic acid and octanoic acid.

By selecting more than one acid for example in which the acids have different n values, the size of the microparticle may be tailored. A longer hydrophobic portion connecting the acid groups suitably provides a larger microparticle. For example where n is 8, sebacic acid, a particle of size 2,6 microns may be obtained and where n is 11, brassylic acid, a particle of size 3,0 microns may be obtained.

When the bis-aliphatic acid is contacted with the aid of a solvent soluble organic base, microparticles are formed spontaneously.

Suitably, the organic base combines with the bis-acid moieties such that the combination of the two components comprises two separate hydrophilic or ionic head regions connected by a hydrophobic region. Without wishing to be bound by theory, it is believed that the hydrophobic regions and hydrophilic regions of adjacent bis-acids with organic base align to form micelles and lead to self-assembly of the microparticles of the invention. Preferably, the microparticle comprises a multi-lamellar structure in which further molecules comprising the bis-acids with the organic base, align with the hydrophilic head of another bis-acid/organic base so as to form a multi-lamellar structure.

The organic base may be selected from a range of bases which, together with the bis-acid forms a self-assembling microparticle. Preferably, the organic base comprises an amine, suitably an aliphatic amine or an aromatic amine having a basic character or other nitrogen-containing base. Examples of suitable organic bases include alkylated amines and polyamines including amines having one or two C₁₋₄ N-alkyl -groups, for example methylated amines. Examples of preferred amines include N-methylmorpholine, 4-methylmorpholine (NMM), N,N-dimethylaminoethanol (DMAE), 4-dimethylaminopyridine (DMAP), imidazole or 1-methylamidazole, poly(diallyldimethylammonium chloride) (PDAC), didecyldimethylammonium chloride (DDAC) and dodecyldipropylenetriamine (DDPT).

In preferred embodiments, the acid is suitably one or more of brassylic acid, sebacic acid and azelaic acid in combination with a base selected from methylmorpholine (NMM), N,N-dimethylaminoethanol (DMAE), 4-dimethylaminopyridine (DMAP), imidazole, 1-methylamidazole, poly(diallyldimethylammonium chloride) (PDAC), didecyldimethylammonium chloride (DDAC) and dodecyldipropylenetriamine (DDPT).

Preferred examples include microparticles comprising brassylic acid and PDAC, brassylic acid and DDAC, brassylic acid and DDPT, sebacic acid and NMM, poly epsilon lysine in combination with one or more of sebacic acid, brassylic acid and azelaic acid.

We have found that microparticles according to the invention comprising amines having antimicrobial properties are particularly suited for use as antimicrobial compositions and biocides. The level of antimicrobial activity of the base may be higher when in the form of a self-assembled microparticle according to the invention as compared to when in a conventional formulation.

According to a further aspect the invention provides an antimicrobial composition comprising a self-assembled microparticle according to the invention as defined herein.

The invention also provides the use of a self-assembled microparticle according to the invention as defined herein and an antimicrobial base having a higher level of antimicrobial activity than the antimicrobial base when not in the form of a self-assembled microparticle.

Suitably, providing the antimicrobial base in a self-assembled microparticle increases the antimicrobial activity and provides at least a 2 log reduction of bacterial load, preferably at least a 4 log reduction of bacterial load and desirably at least a 5 log reduction of bacterial load.

The acid and base are suitably combined in relative quantities such that the molar ratio of acid groups in the acid to basic groups in the base is approximately stoichiometriuc such that self-assembled microparticles form. The molar quantity of acid groups to base groups may be less or more than stoichiometric provided the self-assembled particles form. Where the ratio of acid groups to base groups is too low or too high, -assembled particles do not form as the excess component disrupts structure of the acid and base. The ratio of acid groups to basic groups that allow formation of the self-assembled particle will vary depending on the particular acid and particular base.

The skilled person will be able to determine whether a self-assembled particle is formed by observing under a microscope with magnification at a level to visually observe particles for example at 40x magnification. The relative quantities of the acid and base will be able to be modified to determine the minimum and maximum ratio of the components at which microparticles form. Acids having longer chains may provide microparticles which are more stable than microparticles (with the same base and same molar ratio) comprising an acid having a shorter chain. The greater stability may allow a lower level of acid to be employed and a lower ratio of acid groups to basic groups may still allow a microparticle to form.

Suitably, the ratio of acid groups to basic groups in the acid and base is from 0.7 to 1.3:1, more preferably 0.8 to 1.2:1 and desirably 0.9 to 1.1:1. Sebacic acid and brassylic acid are examples of preferred acids. Suitably a microparticle comprising sebacic acid with a base has a ratio of sebacic acid to base of 0.85 to 1.15:1. A microparticle comprising brassylic acid with a base has a ratio of brassylic acid to base of 0.8 to 1.2:1. In a preferred embodiment, the acid and base are present at levels to provide a molar ratio of acid groups to basic groups of 1:1.

In a second aspect, the invention provides a macroporous material formed by contacting self-assembled microparticles according to the invention under conditions such as to form a macroporous material. The macroporous material is suitably formed by cross-linking the microparticles.

The organic base may be reactive so as to enable cross-linking of the self-assembled microparticles for form a macroporous material. The organic base need not be reactive in which case it may suitably be displaced by another reactive species to allow subsequent cross-linking to form a macroporous material. The solvent soluble organic base can be displaced by addition of a reactive species including, but not limited to, amine containing organic components. The amine suitably allows cross-linking of the microparticles by amide bond formation. In the preferred embodiment the amine containing organic component is a polymeric amine including but not limited to a peptide, protein, polyallylamine, polyethyleneimine and other polyamines.

Examples of suitable amines and polyamines include ethylenediamine, poly-e-lysine, polyallylamine, polyethyleneimine, aminopropyltrialkoxysilanes, 3-(2-aminoethylamino)propyltrimethoxysilane, N-(3-(trimethoxysilyl)-propyl)diethyenetriamine.

In formation of the microparticle or macroporous material the above aforementioned bis acids may be mixed in any proportions. In addition, the reactive amines may also be mixed.

Suitably, the microparticles or macroporous materials comprises functional components, tailored according to the intended use. For example the addition of ethylenediamine tetra acetic acid imparts metal chelating properties.

In another embodiment, polyethylene imines may be employed in binding or as a support structure in synthesis or manipulation for example sequencing of nucleic acids including DNA, RNA.

Alkoxysilanes may be employed and may form a silica shell in the lamellar layers of the microparticle.

In another embodiment the active site of a specific enzyme can be incorporated into a peptide within the particles to allow for controlled release of an active agent. For example, the cleavage site of a wound based metallino-protease could be incorporated in wound care based materials to allow controlled release of an antibacterial agent.

In another application, the microparticles of the invention may be employed to form a macrostructure for the growth of bone cells. Suitably, the microparticle is combined with hydroxyapatite preferably in crystalline form in order to attract bone cells and promote bone cell culture.

Many commercially available wound dressings now incorporate animal derived collagen which has been shown to improve wound healing. The microparticles and macroporous materials of this invention are useful in replacing the animal derived collagen in wound dressings. The macroporous materials of this invention have been shown to mimic the biological and physical properties of collagen in tissue repair. However, the components of these materials are not burdened by the growing ethical and religious concerns over the use of animal based materials in human healthcare.

A self-assembled microparticle or macroporous material according to the invention may also comprise a functional material supported by the polymer. Examples of suitable functional materials include a catalyst, an initiator species for peptide synthesis or oligonucleotide synthesis, a pharmaceutical active, an agrochemical active, a macromolecule, an enzyme, a nucleic acid sequence and a protein.

The invention is particularly useful in supporting precious metal catalysts, for example palladium catalysts. A particular advantageous example is palladium.

The invention provides in a further aspect a method for producing a self-assembled microparticle or macroporous material as defined in the claims.

Suitably the polymerisation and cross-linking is initiated by processes known to those skilled in the art. For example, self-assembled microparticle or macroporous material prepared in water with an amine containing component can be cross-linked using a water soluble carbodiimide.

The self-assembled microparticle or macroporous material of the invention may be used in any chemical or physical process in which a solid support is used.

The self-assembled microparticle or macroporous material may be employed in applications involving electro-conducting and light emitting polymers. The particulate support containing light emitting polymers may be arranged on display panels.

The self-assembled microparticle or macroporous material is particularly useful for solid phase synthesis of an organic species, particularly macromolecules. In a preferred embodiment the self-assembled microparticle or macroporous material may be employed in the synthesis of peptides, oligonucleotides or oligosaccharides.

In solid phase synthesis, the process for using solid polymeric particles in applications such as peptide synthesis typically involves suspending the particles in the appropriate solvent above a porous filter plate and stirring the particles gently so as not to mechanically damage the particles. The manufacturing process for the particles often generates fines that cause blockages in the filter plate leading slow filtration or the need to replace or clean the filter. In addition, the stirring of solid particles may cause fracture leading to generation of fines that exaggerate the problems of filter blockage. In the pharmaceutical and associated industries strict quality regulations under current good manufacturing process (cGMP) require that the filter plate is replaced following each batch of product in order to avoid contamination of subsequent batches with material dislodged from the filter plate.

Suitably, the self-assembled microparticle or macroporous material according to the invention is substantially mono-disperse. That is the material has particles which are all substantially the same size. Monodisperse microparticles or macroporous materials advantageously simplify solid phase synthesis.

The invention further provides for the use of a self-assembled microparticle or macroporous material according to the invention as a solid phase in a chromatographic process.

Conventionally, chromatography columns are generally packed by preparing a slurry of the particles, or stationary phase in a suitable solvent and transferring this into the column with the lower column filter plate present. Uneven settling of the bed due to broad particle size distribution in chromatography columns can cause uneven and even cracked stationary phase beds resulting in poor and irreproducible separations. Columns often have to be emptied and repacked several times to achieve the required performance. This can be laborious and leads to down time which is a particular disadvantage in process scale operations.

The virtually monodispersed nature of the self-assembled microparticles in a preferred embodiment of the invention allows preparation of the slurry and transfer of the slurry to the column to form a more uniform bed. Alternatively the macroporous material formed by collision of the self-assembled microparticle can be used to prepare a monolithic chromatography column.

In another embodiment the interstitial spaces between the particles in a monolith may be filled with a different component such as a cell culture nutrient for example. In this example the cells may be cultured on the surface of the self-assembled macroporous material. In this example the self-assembled macroporous material is often referred to as a scaffold for three dimensional cell culture. The materials described herein will therefore have applications in regenerative medicine, 3D cell culture and wound care.

The self-assembled microparticles and macroporous material of the invention are also useful for solid phase extraction to remove species from a liquor which is contacted with the support, whether in batch form or as a flow over the support, for example ion extraction and ion exchange. Solid phase extraction is typically performed in columns or in systems with filter plates for separation of the solid phase from the mixture under extraction. The problems observed for solid phase synthesis and chromatography referred to herein may similarly be observed with solid phase extraction. The self-assembled microparticles and macroporous material of the invention provides similar advantages as afforded in chromatography and solid phase synthesis.

The self-assembled microparticles and macroporous material of the invention may be used to immobilise species including antibodies, oligonucleotides, enzymes or fluors and may be positioned in an array, with each support assaying a different component of a solution. Self-assembled microparticles and macroporous material having ligands covalently attached to their surface, may be employed as 'wells'. Specific binding of a target ligand such as antigen or complimentary DNA or RNA sequence may then be detected using established methods.

The self-assembled microparticles and macroporous material of the invention also may be employed to immobilise a biocatalyst. Biocatalysts are often used in columns or in systems with filter plates for separation of the solid phase from the mixture under extraction. The problems observed for solid phase synthesis and chromatography referred to herein may similarly be observed with solid phase extraction.

The self-assembled microparticles and macroporous material of the invention is especially useful in immobilising species including solid phase reagents, metal and other catalysts, bio-catalysts, enzymes, proteins, antibodies including polyclonal and monoclonal antibodies, whole cells and polymers. The invention is particularly advantageous in supporting enzymes, for example the lipase Cal B, commonly used in detergents and personal care products.

The present invention is also especially useful in the immobilisation of affinity ligands such as Protein A.

In a further application, the particulate support of the invention may also be used in chemocatalysis, for example by immobilizing transition metal catalysts and ligands.

In yet a further application, the present invention may be used in cell culture. Mass culture of animal cell lines is fundamental to the manufacture of viral vaccines, biopharmaceuticals and many products of biotechnology. Biological products produced by recombinant DNA technology in animal cell cultures include enzymes, synthetic hormones, immunobiologicals (monoclonal antibodies, interleukins, and lymphokines) and anticancer agents. Many simpler proteins can be produced using rDNA in bacterial cultures; more complex proteins that are glycosylated (carbohydrate-modified) currently must be made in animal cells. An important example of such a complex protein is the hormone erythropoietin. The cost of growing mammalian cell cultures is high, so companies are constantly looking to improve techniques.

Cells can be grown in suspension or as adherent cultures. However, adherent cells require a surface, which may be coated with extracellular matrix components to increase adhesion properties and provide other signals needed for growth and differentiation. Generally cells derived from solid tissues are adherent. Organotypic culture involves growing cells in a three-dimensional environment as opposed to two-dimensional culture dishes. This 3D culture system is biochemically and physiologically more similar to in vivo tissue, but is technically challenging to maintain because of many factors (e.g. diffusion).

In a further aspect, the invention provides for the use of self-assembled microparticles and macroporous material according to the invention to culture cells on the surface of the support. Suitably, stem cells may be cultured on the self-assembled microparticles and macroporous material of the invention to reduce uncontrolled differentiation and to control desired differentiation. The handling characteristics of the self-assembled microparticles and macroporous material and high utilisation of surface area of the support is advantageous in this application.

The invention is particular useful in medical diagnostic tests such as immunoassay. Accordingly the invention further provides medical diagnostics for detecting the presence of a compound comprising self-assembled microparticles and/or macroporous material according to the invention and a functional material such as an enzyme, for example horseradish peroxidase, supported by the polymer in the support for selectively reacting with or binding to the compound to be detected.

The self-assembled microparticles and/or macroporous material of the present invention may be used in separation processes, for example magnetic separation, flow cytometry, drug delivery and in a wide range of fields including the detergent, agrochemical and personal care fields.

Many medical diagnostics rely upon solid supports to immobilise various diagnostic ligands. The self-assembled microparticles and/or macroporous material of the present invention may be used in a medical diagnostic procedure where physical separation of the solid phase through a liquid phase.

In a further application, the self-assembled microparticles and/or macroporous material may be used as an absorbent. In this application, it is especially advantageous if the support contains an inert, absorbent material bound to the self-assembled microparticles and/or macroporous material. The self-assembled microparticles and/or macroporous material may be used to absorb household spillages, for example tea, coffee and wine, or may be used in larger-scale applications for example, to absorb oil from spillages. The absorbent support may be used to absorb the spillage and then physically removed or, in the case of oil spillage in a body of water, effectively trap the oil and retain the oil in a retained mass for collection and disposal.

The self-assembled microparticles and/or macroporous material of the invention may be used as a carrier to carry a compound which is to be released over a period of time, for example a pharmaceutical or agrochemical compound or composition. This use provides a means of tailoring a dosage regime of the compound according to the loading of the compound in the support. In the case of a pharmaceutical, this may be advantageous in assisting the correct dosage of an active, for example with continuous slow release rather than requiring a patient to take periodic large doses, for example in chemotherapy. In the case of over the counter drugs the microparticles could be used to deliver nasal decongestants, antiseptics and anti-inflammatory aids. In some cases microparticles have been used to deliver natural oils such as peppermint oil and lavender oil as anti-snoring aids.

Apart from inhalation the self-assembled microparticles can be used, in suitable form, for intravenous drug delivery or for delivery of vaccines.

The microparticles of the invention or macroporous materials of the invention may be formulated into a composition for a wide-range of uses including a composition for use in personal care products for example a topical composition and an oral composition, homecare products and medical products for example wound treatment products. Examples of personal care products include a handwash, handscrub, cream, deodorant, shampoo and conditioner. Examples of homecare products include antimicrobial products, surface sprays cleaners, detergents and the like. The topical composition may comprise a functional material suitable for topical delivery.

The disclosure is illustrated by the following non-limiting examples None of the examples is according to the invention.

### Example 1 - Preparation of self-assembled microparticles

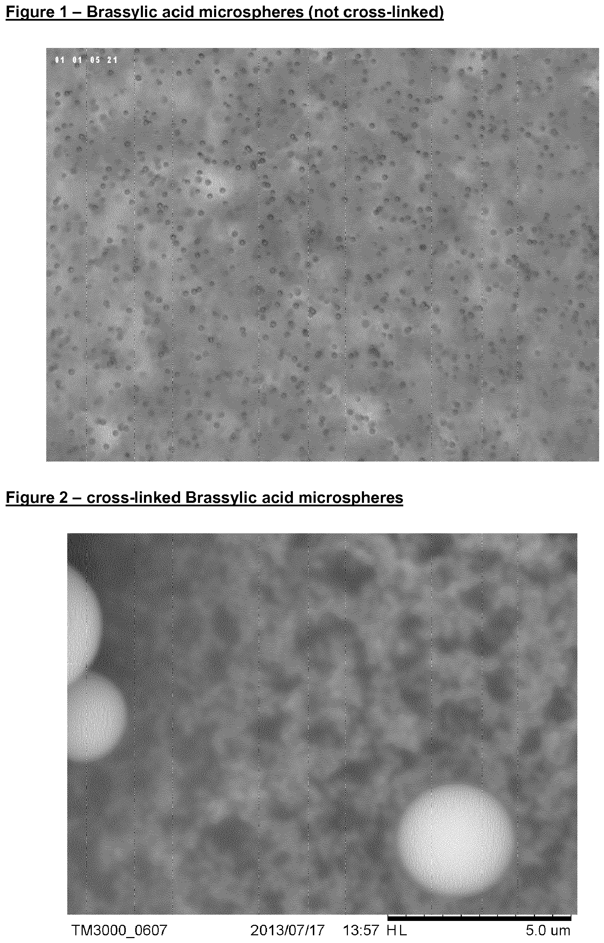

Brassylic acid (1.54g, 6.31mmol) and 4-dimethylaminopyridine (DMAP, 1.54g, 12.62mmol) were dissolved in water (10cm³) and a sample placed on a microscope. Almost monodispersed spherical entities of ~3µm diameter were observed (Figure 1).

### Example 2 - Preparation of self-assembled microparticles

Brassylic acid (1.54g, 6.31mmol) and dimethylaminoethanol (DMAE, 1.12g, 12.62mmol) were dissolved in water (10cm³) and a sample placed on a microscope. Almost monodispersed spherical entities of ∼3µm diameter were observed.

### Example 3 - Preparation of self-assembled microparticles

Brassylic acid (1.54g, 6.31mmol) and 4-methylmorpholine (NMM, 1.275g, 12.62mmol) were dissolved in water (10cm³) and a sample placed on a microscope. Almost monodispersed spherical entities of ~3µm diameter were observed.

### Example 4 - Preparation of self-assembled microparticles

The above dicarboxylic acid dissolution experiments were also carried out using a range of acids and a range of water soluble organic bases. Some of the combinations tested are listed below. The combinations had an acid group to basic group molar ratio of 0.9 to 1.1:1. All of these combinations formed the spherical entities as described in

### Example 1.

Pimelic acid plus NMM
Suberic acid plus NMM
Azelaic acid plus NMM
Sebacic acid plus NMM
Sebacic acid plus DMAP
Sebacic acid plus DMAE
Sebacic acid plus imidazole
Dodecanedioic acid plus NMM
Dodecanedioic acid plus DMAP
Dodecanedioic acid plus DMAE
C36 dimer acid plus NMM

### Example 5 - Preparation of cross-linked self-assembled microparticles

Brassylic acid (1.54g, 6.31mmol) and 4-dimethylaminopyridine (DMAP, 1.54g, 12.62mmol) were dissolved in water (10cm³) and a sample placed on a microscope. Almost monodispersed spherical entities of ~3µm diameter were observed (Figure 1). Poly-epsilon-lysine (PeK) (2g, 12.04mmol of NH₂) was dissolved in water (10cm³) and added to the above solution of Brassylic acid/DMAP microspheres. The mixture was filtered through a 0.45µm membrane and a sample placed on a microscope. Microspheres of ~3µm diameter were still present. This solution was diluted with water to 100cm³. N-(3-Dimethylaminopropyl)-N'-ethylcarbodiimide hydrochloride (EDCI)(4.6g, 2.4mmol) and HONSu (1.38g,1.2mmol) were dissolved in water (10cm³) and added to the above solution. The cross-linking reaction was left overnight, the resultant particles washed by tangential flow filtration (TFF) and recovered by lyophilisation (yield 2.35g). Figure 2 shows a scanning electron micrograph of the resultant microspheres.

### Example 6 - Preparation of cross-linked self-assembled microparticles containing Protoporphyrin IX, Heme B

Brassylic acid (0.734g, 3.3mmol) and 4-dimethylaminopyridine (DMAP, 0.734g, 6.6mmol) were dissolved in water (10cm³) and a sample placed on a microscope. Almost monodispersed spherical entities of ~3µm diameter were observed (Figure 1). Poly-epsilon-lysine (PeK) (1g, 6.02mmol of NH₂) was dissolved in water (10cm³) and added to the above solution of Brassylic acid/DMAP microspheres. The mixture was filtered through a 0.45µm membrane and a sample placed on a microscope. Microspheres of ~3µm diameter were still present. This solution was diluted with a saturated solution of Heme B (50cm³). N-(3-Dimethylaminopropyl)-N'-ethylcarbodiimide hydrochloride (EDCI)(2.3g, 1.2mmol) and HONSu (0.7g,0.6mmol) were dissolved in water (5cm³) and added to the above solution. The cross-linking reaction was left overnight, the resultant particles washed by tangential flow filtration (TFF) and recovered by lyophilisation (yield 0.93g).

### Example 7 - Preparation of cross-linked self-assembled microparticles

Sebacic acid (0.619g, 6.12mmol) and NMM (0.62g, 6.12mmol) were dissolved in water (10cm³) and a sample placed on a microscope. Almost monodispersed spherical entities of ~2.5µm diameter were observed.

Poly-epsilon-lysine (PeK) (1g, 5.83mmol of NH₂) was dissolved in water (10cm³) and added to the above solution of Sebacic acid/NMM microspheres. The mixture was filtered through a 0.45µm membrane and a sample placed on a microscope. Microspheres of ~2.5µm diameter were still present. This solution was diluted with water to 50cm³. EDCI (2.24g, 11.7mmol) and HONSu (2.0g, 17.4mmol) were dissolved in water (10cm³) and added to the above solution. The cross-linking reaction was left overnight, the resultant particles washed by TFF and recovered by lyophilisation.

### Example 8 - Preparation of cross-linked self-assembled microparticles

Sebacic acid (5.06g, 25mmol) and imidazole (3.4g, 50mmol) were dissolved in water (50cm³) and a sample placed on a microscope. Almost monodispersed spherical entities of ~2.5µm diameter were observed.

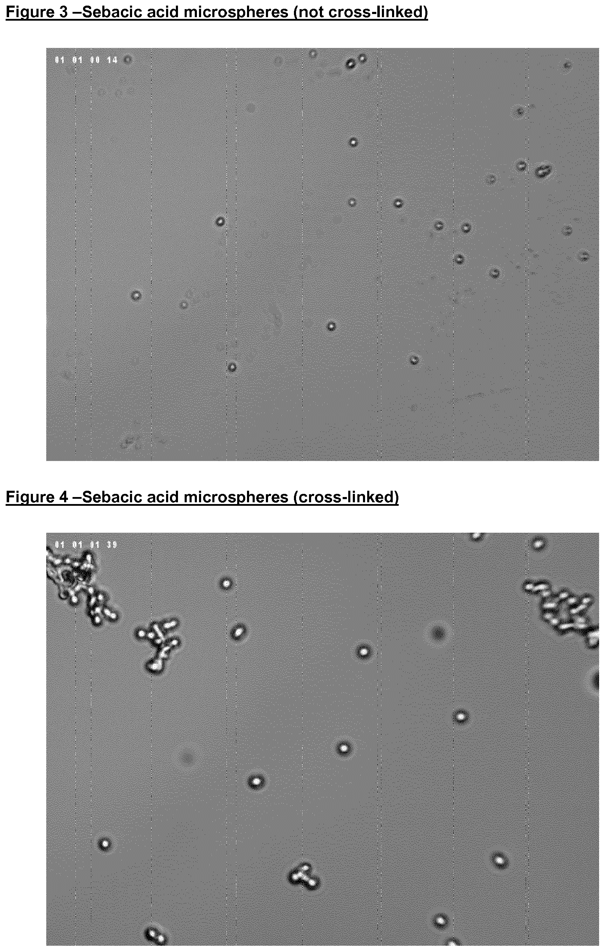

Poly-epsilon-lysine (PeK) (8.576g, 50mmol of NH₂) was dissolved in water (50cm³) and added to the above solution of Sebacic acid/imidazole microspheres. The mixture was filtered through a 0.45µm membrane and a sample placed on a microscope. Microspheres of ~2.5µm diameter were still present (Figure 3). This solution was diluted with water to 500cm³. EDCI (4.8g, 25mmol) was dissolved in water (20cm³) and added to the above solution. The cross-linking reaction was left for 1h then a further 25mmol of EDCI added before leaving overnight. The resultant particles washed with water by decantation and recovered by lyophilisation (Figure 4).

### Example 9 - Preparation of cross-linked self-assembled microparticles

Sebacic acid (5g, 24.7mmol) and (3-Aminopropyl)trimethoxysilane (8.42g, 46.9mmol) were dissolved in water (50cm³) and a sample placed on a microscope. Almost monodispersed spherical entities of ~2.5µm diameter were observed.

The mixture was left overnight then acidified with concentrated hydrochloric acid. The addition of hydrochloric acid led to the formation of silica within the particles creating a Sebacic acid/silica composite.

### Example 10 - Preparation of cross-linked self-assembled microparticles

Sebacic acid (5g, 24.7mmol) and N-[3-(Trimethoxysilyl)propyl]ethylenediamine (5.77g, 51.9mmol of amine) were dissolved in water (50cm³) and a sample placed on a microscope. Almost monodispersed spherical entities of ~2.5µm diameter were observed.

This solution was diluted with water to 500cm³. EDCI (20g, 104mmol) was dissolved in water (100cm³) and added to the above solution. The mixture was left overnight then acidified with concentrated hydrochloric acid. The addition of hydrochloric acid led to the formation of silica within the particles creating a Sebacic acid/silica composite.

### Example 11 - Preparation of cross-linked self-assembled microparticles

Sebacic acid (5g, 24.7mmol) and N1-(3-Trimethoxysilylpropyl)diethylenetriamine (4.37g, 46.9mmol of amine) were dissolved in water (50cm³) and a sample placed on a microscope. Almost monodispersed spherical entities of ~2.5µm diameter were observed.

This solution was diluted with water to 500cm³. EDCI (20g, 104mmol) was dissolved in water (100cm³) and added to the above solution. The mixture was left overnight then acidified with concentrated hydrochloric acid. The addition of hydrochloric acid led to the formation of silica within the particles creating a Sebacic acid/silica composite.

### Example 12 - Preparation of a self-assembled macroporous cross-linked sheet

Sebacic acid (0.619g, 6.12mmol) and NMM (0.62g, 6.12mmol) were dissolved in water (10cm³) and a sample placed on a microscope. Almost monodispersed spherical entities of ~2.5µm diameter were observed.

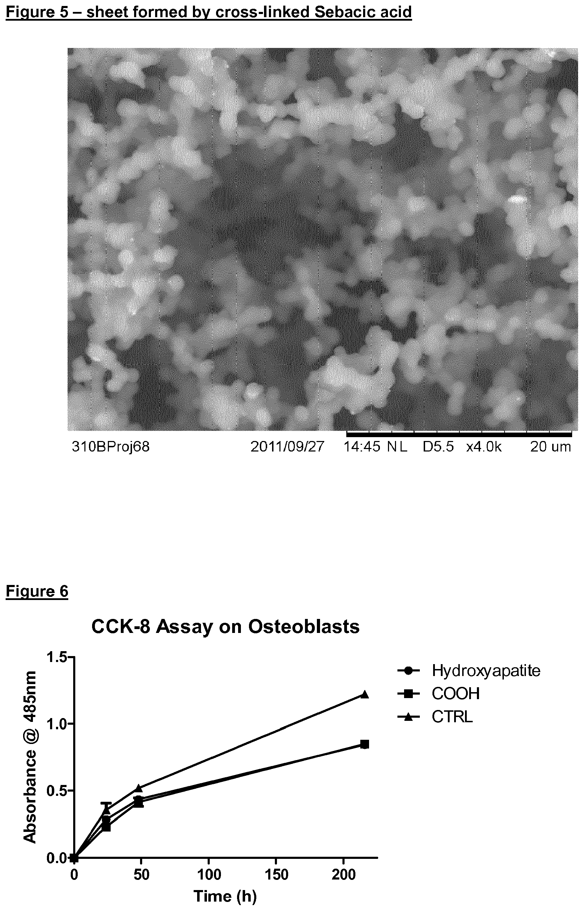

Poly-epsilon-lysine (PeK) (1g, 5.83mmol of NH₂) was dissolved in water (10cm³) and added to the above solution of Sebacic acid/NMM microspheres. The mixture was filtered through a 0.45µm membrane and a sample placed on a microscope. Microspheres of ~2.5µm diameter were still present. EDCI (2.24g, 11.7mmol) and HONSu (2.0g, 17.4mmol) were dissolved in water (10cm³) and added to the above solution. The cross-linking reaction was left overnight, the resultant sheets washed with water and dried by lyophilisation. The SEM shown in Figure 5 clearly demonstrates the fused microsphere structure of the macroporous polymer formed.

### Example 13 - Preparation of a self-assembled macroporous cross-linked sheet

(12-Phosphonododecyl)phosphonic acid (330mg, 1mmol) and NMM (404mg, 4mmol were dissolved in water. A sample placed on a microscope confirmed the presence of virtually monodispersed microspheres. PeK (343mg, 2mmol NH₂) was dissolved in water (10cm³) and added to the bis-phosphonic acid solution prepared above. Microspheres were still present at this stage. EDCI (1.15g, 6mmol) dissolved in water (10cm³) was added and the mixture immediately poured into a tray. Again microspheres were still present at this stage. A sheet formed after ~2h which was washed thoroughly with water. The final sheet had a rubbery texture.

### Example 14 - Bone cell culture (example of 3D cell culture)

In this Example a self-assembled macroporous sheet as produced in Example 12 was produced. A further product comprising the sheet of Example 12 together with about 10% by weight relative to the scaffold of hydroxyapatite nanoparticle available from Sigma Aldrich under catalogue number 702153 and the cytocompatibility was tested.

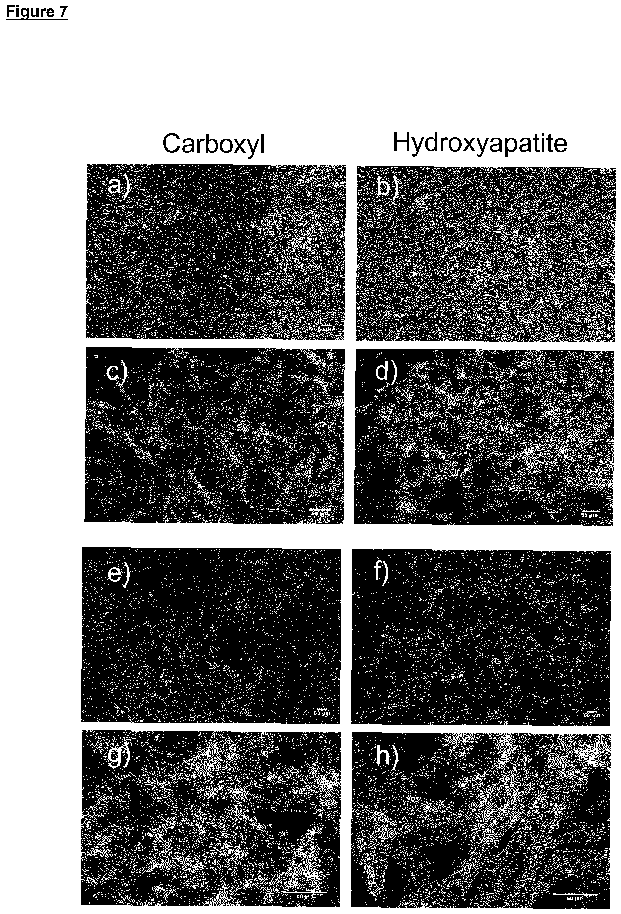

The 9 day growth curve of osteoblasts bone cells demonstrated there was no significant difference between cell viability during the early stages of the culture period when compared to the tissue culture plastic control. There was no significant difference in cell viability between osteoblast cultured on either the carboxyl or hydroxyapatite coated scaffolds. Cellular interaction with the 3D scaffold became more apparent at x40 magnification and actin filament staining highlighted adhesion points where cells had anchored to the 3D scaffold as shown in Figure 7.

Figure 7 shows osteoblasts cultured on a carboxyl functionalized and hydroxyapatite coated 3D scaffolds, a + b) cultured for 48 h at x10 magnification, c + d) cultured for 48 h at ×40 magnification, e + f) cultured for 7 days at ×10 magnification, g + h) cultured for 7 days at ×20 magnification.

Osteoblasts continued to survive and interact with the scaffold for the rest of the culture period. These results demionstrate that the 3D scaffold with/without the hydroxyapatite coating supports osteoblast growth.

### Osteoblast Proliferation

Osteoblasts were seeded at a density of 1 × 10⁵ cells on 24 well inserts and cultured under standard tissue culture conditions of 37°C and 5% CO₂. Dulbecco's Modified Eagle Medium (DMEM) (high glucose + 2 mM glutamine) media containing 10% FCS with fungizone and pen/strep supplementation was used. Cellular F-actin was stained with FITC conjugated Phalloidin and then counter-stained with the nuclear stain, Hoechst 33342. Images were taken at time points 24 h, 48 h, and 7 days using a Nikon Eclipse Ti-E phase contrast microscope (Nikon, Tokyo, Japan) (Figure 6). Figure 6: Metabolic activity assay (CCK-8) of osteoblasts cultured on a carboxyl and hydroxyapatite coated 3D scaffold over a 9 day culture period.

### Cell Proliferation Assay

A CCK-8 assay kit (Dojindo Laboratories, Kumamoto, Japan) was used to monitor cell proliferation at various time periods during the course of a 9 day cell culture period. A standard curve to determine cell number was carried out following the manufacturer's guidelines. Cells were incubated under standard culture conditions after seeding scaffolds with an initial density of 5 × 10⁴ cells. At each time-point CCK-8 solution (50 mm³) was added to the media (500 mm³) in each well. The cells were then incubated under standard culture conditions for 2 h. An aliquot (3 x 100 mm³) of solution from each well was pipetted into labelled wells in a 96-well plate. Suitable controls were also used. The absorbance was read at 485 nm using a FLUOstar Optima plate reader (BMG Labtech, Ortenberg, Germany) with a background reading at 600 nm and the results recorded.

### Example 15 - Biocide formulations

Biocides for personal care, cosmetics, home care and general disinfection are currently limited by the time they stay in contact with the surface to be treated due to abrasion. For example, surface sprays of the type used for disinfection in hospitals have a limited active lifetime and consequently reduced activity against hospital infections such as MRSA, *p.auregenosa* and *c.difficile.* In addition, some surface sprays contain organic solvents such as isopropanol or non-biodegradable components such as silicone oils to reduce abrasive removal of the biocides.

Cationic and amphoteric biocides such as quaternary ammonium compounds act against pathogens by solubilising the cell membrane, resulting in cell lysis and death. There are many biocides used commercially for disinfection which include chlorhexidine, benzalkonium chloride, climbazole, didecyldimethylammonium chloride, dodecyldipropylenetriamine, which are cationic compounds. Additionally some biocides are polymeric cationic compounds such as Poly(diallyldimethylammonium chloride). These compounds can be readily formulated into spherical microparticles using the technology described herein, which will allow for reduced abrasive removal on surfaces, skin and hair; potentially allowing for controlled release of the biocide. Additionally, biocides containing multiple cationic compounds in the same microparticle are possible and may provide formulations that can be tailored and targeted to specific applications where the source of infection is well defined.

The samples produced were as follows:

### Poly(diallyldimethylammonium chloride) (PDAC) SpheriSomes

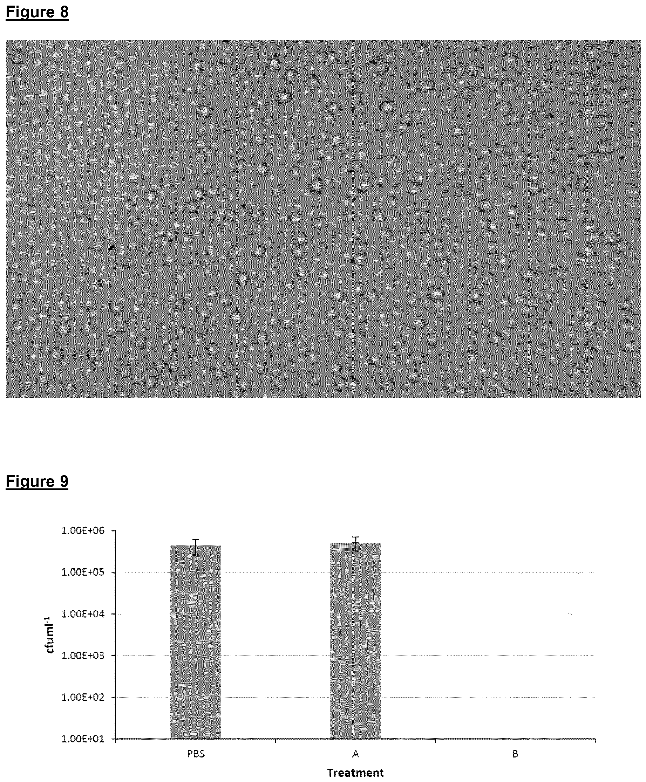

PDAC (1.615g, 10mmol) was dissolved in water (50cm³) and NaOH (0.4g, 10mmol) added. Brassylic acid (1.22g, 5mmol) was added to this solution and allowed to dissolve overnight. This appeared to be a clear solution but was confirmed to be a suspension of ~3µm microparticles and a novel formulation of PDAC when observed under the microscope and the results are shown in Figure 8 shows PDAC-Brassylic acid microparticle formulation.

### Didecyldimethylammonium chloride (DDAC)

DDAC (9.04cm³ of 40%w/v solution, 10mmol) was diluted with water to 50cm³ and NaOH (0.4g, 10mmol) added. Brassylic acid (1.22g, 5mmol) was added to this solution and allowed to dissolve overnight. This appeared to be a hazy solution but was confirmed to be a suspension of ~3µm microparticles and a novel formulation of DDAC when observed under the microscope.

### Dodecyldipropylenetriamine (DDPT)

DDPT (9.97cm³ of 30%w/v solution, 10mmol) was diluted with water to 50cm³ and Brassylic acid (3.66g, 15mmol) was added to this solution and allowed to dissolve overnight. This appeared to be a clear solution but was confirmed to be a suspension of ~3µm microparticles and a novel formulation of DDPT when observed under the microscope.

### Example 16 - Antimicrobial Wound Dressings

The hydrophilic nature of the porous polymer formed by collision of the biscarboxy fatty acid microparticles is advantageous in for absorbent wound dressings. When the biscarboxy fatty acids are combined with poly-ε-lysine and cross-linked to form such a porous matrix the natural antimicrobial activity of the components of the wound dressing can be retained and enhanced when necessary. In cationic form, where there is an excess of poly-ε-lysine over the fatty acids the materials have been shown to retain the characteristics of the food preservative providing novel antimicrobial wound dressings. The porous nature of the material will allow for improved skin repair as a 3D scaffold combined with a cationic nature capable of destroying microbial biofilms.

The anti-biofilm capability of a cationic wound dressing was assessed using a mixed species CDC reactor model. The product of Example 13 was employed in these experiments.

Two mixed species biofilms were prepared as shown below and tested against PBS and a control anionic dressing.

### Multi species biofilm 1

*Staphylococcus aureus* NCTC 8325
*Pseudomonas aeruginosa* NCIMB 10434
*Acinetobacter baumannii* ATCC 19606
*Staphylococcus epidermidis*

### Multi species biofilm 2

*Staphylococcus aureus* NCTC 8325
MRSA
VRE *faecalis* NCTC 12201
*Candida albicans* ATCC MYA-2876 SC5313
*Escherichia coli* NCTC 12923 Page 3 of 6 *DOT 202 (03)*

### Preparation of mixed inoculum 1

Twenty-four hour cultures of *Staphylococcus aureus, Pseudomonas aeruginosa, Acinetobacter baumannii,* and *Staphylococcus epidermidis* were harvested from appropriate agar plate using a sterile swab and suspended in 20cm³ of Tryptone Soya Broth (TSB). The mixed species suspension was diluted in TSB to give an overall concentration of 107 ± 5 x106 cfuml-1 and used as the inoculum for the CDC reactor. The CDC reactor was incubated for 72 hours at 37°C with shaking at 50rpm in order to encourage biofilm growth.

### Preparation of mixed inoculum 2

Twenty-four hour cultures of *Staphylococcus aureus, Methicillin-resistant staphylococcus aureus, Vancomycin-resistant Enterococcus, Candida albicans* and *Escherichia coli* were harvested from appropriate agar plate using a sterile swab and suspended in 20cm³ of TSB. The mixed species suspension was diluted in TSB to give an overall concentration of 107 ± 5 ×106 cfuml-1 and used as the inoculum for the CDC reactor. The CDC reactor was incubated for 72 hours at 37°C with shaking at 50rpm in order to encourage biofilm growth.

### Biofilm treatment

After incubation the test coupons were removed from the CDC reactor and washed 3 times in sterile phosphate buffered saline (PBS) in order to remove planktonic cells. The washed coupons were then treated by sandwiching the coupon between two discs of the wound dressing material. The dressings were activated prior to testing by the addition of 400mm³ PBS + 1% TSB to each disc. Control coupons were submerged in 1cm³ of PBS + 1% TSB. All samples were tested in triplicate. Following the 24 hour treatment period, the coupons were placed in 1cm³ PBS and sonicated for 15 minutes in order to recover any viable microorganisms attached to the coupons. Recovered microorganisms were quantified using serial dilutions and spread plates.

### Mixed Inoculum 1

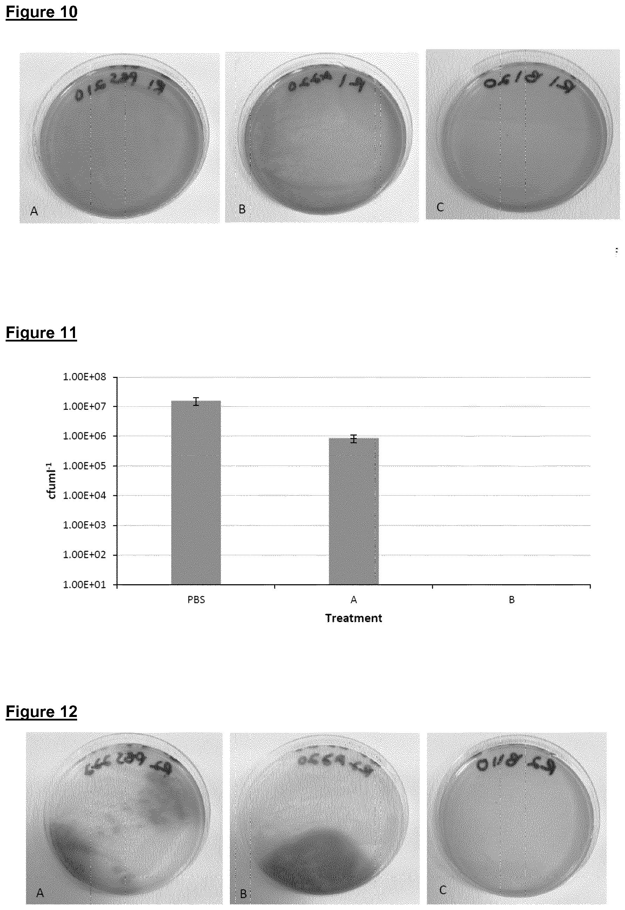

Following treatment with the control dressing (A), bacterial recovery was similar to PBS only treatment controls as shown in Figure 9. No viable organisms were recovered from coupons treated with cationic dressing (B). This represents a greater than 5 log reduction compared to PBS treated controls. The surviving organism post treatment were predominantly *Pseudomonas aeruginosa* (Figure 10).

### Mixed Inoculum 2

Treatment with control dressing (A) resulted in a 1.27 log reduction in the number of viable bacteria recovered compared to the PBS treated controls. No viable organisms were recovered from coupons treated with cationic dressing (B). This represents a greater than 7 log reduction compared to the PBS treated controls (Figure 11). Surviving organisms were mixed species (Figure 12).

## Claims

1. A self-assembled microparticle comprising an acid of general formula HOOC-(CH₂)ₙ-COOH wherein n is at least 5 and not more than 40 and an organic base in which the molar ratio of acid groups to basic groups in the acid and base is from 0.6 to 1.4:1, the microparticle being obtainable by a process comprising contacting the said acid and organic base in aqueous solution, wherein the acid is insoluble in the aqueous solution and the organic base is soluble in the aqueous solution wherein the microparticle comprises a multi-lamellar structure.

2. A microparticle according to any one of the preceding claims wherein the acid comprises brassylic acid, sebacic acid and/or azelaic acid.

3. A microparticle according to any one of the preceding claims wherein the organic base comprises an aliphatic amine or an aromatic amine having a basic character or other nitrogen-containing base.

4. A microparticle according to any one of the preceding claims wherein the organic base comprises one or more of N-methylmorpholine, N,N-dimethylaminoethanol, 4-dimethylaminopyridine, imidazole, 1-methylamidazole poly(diallyldimethylammonium chloride) (PDAC), didecyldimethylammonium chloride (DDAC), dodecyldipropylenetriamine (DDPT) and poly epsilon lysine.

5. A microparticle according to any one of the preceding claims wherein the organic base is displaced with another reactive base which is then reacted to form a cross-linked species.

6. A macroporous material comprising micro particles according to any one of the preceding claims, the microparticles being contacted so as to form cross links between the microparticles and thereby form a three dimensional body.

7. Use of a self-assembled microparticle according to any one of claims 1 to 5 or macroporous material according to claim 6 as a macrostructure for three-dimensional cell culture or for use in wound dressings instead of animal derived collagen.

8. Use according to claim 7 for the growth of bone cells.

9. A method for producing a self-assembled microparticle or macroporous material according to any one of claims 1 to 6 in aqueous media comprising contacting the acid of general formula HOOC-(CH₂)ₙ-COOH wherein n is at least 5 and not more than 40 with an organic base in an aqueous medium in which the molar ratio of acid groups to basic groups in the acid and base is from 0.6 to 1.4:1 and the acid is insoluble in the aqueous medium and the organic base is soluble in the aqueous medium.

10. An antimicrobial composition comprising a self-assembled microparticle according to any one of claims 1 to 5.

## Patentansprüche

1. Selbstorganisiertes Mikropartikel, umfassend eine Säure der allgemeinen Formel HOOC-(CH₂)ₙ-COOH, wobei n mindestens 5 und höchstens 40 ist, und eine organische Base, wobei das Molverhältnis von sauren Gruppen zu basischen Gruppen in der Säure und der Base 0,6 bis 1,4:1 beträgt, wobei das Mikropartikel erhältlich ist durch ein Verfahren, umfassend das Inkontaktbringen der Säure und der organischen Base in wässriger Lösung, wobei die Säure in der wässrigen Lösung unlöslich ist und die organische Base in der wässrigen Lösung löslich ist, wobei das Mikropartikel eine multilamellare Struktur umfasst.

2. Mikropartikel nach einem der vorangehenden Ansprüche, wobei die Säure Brassylsäure, Sebacinsäure und/oder Azelainsäure umfasst.

3. Mikropartikel nach einem der vorangehenden Ansprüche, wobei die organische Base ein aliphatisches Amin oder ein aromatisches Amin mit basischem Charakter oder eine andere stickstoffhaltige Base umfasst.

4. Mikropartikel nach einem der vorangehenden Ansprüche, wobei die organische Base eines oder mehrere von N-Methylmorpholin, N,N-Dimethylaminoethanol, 4-Dimethylaminopyridin, Imidazol, 1-Methylimidazol, Poly(diallyldimethyl-ammoniumchlorid) (PDAC), Didecyldimethylammoniumchlorid (DDAC), Dodecyldipropylentriamin (DDPT) und Poly(ε-Lysin) umfasst.

5. Mikropartikel nach einem der vorangehenden Ansprüche, wobei die organische Base ersetzt wird durch eine andere reaktive Base, die dann umgesetzt wird, um eine vernetzte Spezies zu bilden.

6. Makroporöses Material, umfassend Mikropartikel nach einem der vorangehenden Ansprüche, wobei die Mikropartikel in Kontakt gebracht werden, um Vernetzungen zwischen den Mikropartikeln zu bilden und dadurch einen dreidimensionalen Körper zu bilden.

7. Verwendung eines selbstorganisierten Mikropartikels nach einem der Ansprüche 1 bis 5 oder eines makroporösen Materials nach Anspruch 6 als Makrostruktur für die dreidimensionale Zellkultur oder zur Verwendung in Wundauflagen anstelle von Kollagen tierischen Ursprungs.

8. Verwendung nach Anspruch 7 für das Wachstum von Knochenzellen.

9. Verfahren zur Herstellung eines selbstorganisierten Mikropartikels oder eines makroporösen Materials nach einem der Ansprüche 1 bis 6 in wässrigen Medien, umfassend das Inkontaktbringen der Säure der allgemeinen Formel HOOC-(CH₂)ₙ-COOH, wobei n mindestens 5 und höchstens 40 ist, mit einer organischen Base in einem wässrigen Medium, wobei das Molverhältnis von sauren Gruppen zu basischen Gruppen in der Säure und der Base 0,6 bis 1,4:1 beträgt und die Säure in dem wässrigen Medium unlöslich ist und die organische Base in dem wässrigen Medium löslich ist.

10. Antimikrobielle Zusammensetzung, umfassend ein selbstorganisiertes Mikropartikel nach einem der Ansprüche 1 bis 5.

## Revendications

1. Microparticule auto-assemblée comprenant un acide de formule générale HOOC-(CH₂)ₙ-COOH dans laquelle n est supérieur ou égal à 5 et inférieur à 40 et une base organique dans laquelle le rapport molaire des groupes acides sur les groupes basiques de l'acide et la base est compris entre 0,6 et 1,4:1, la microparticule pouvant être obtenue par un procédé comprenant la mise en contact desdits acide et base organique dans une solution aqueuse, dans laquelle l'acide est insoluble dans la solution aqueuse et la base organique est soluble dans la solution aqueuse dans laquelle la microparticule comprend une structure multilamellaire.

2. Microparticule selon l'une quelconque des revendications précédentes dans laquelle l'acide comprend l'acide brassylique, l'acide sébacique et/ou l'acide azélaïque.

3. Microparticule selon l'une quelconque des revendications précédentes dans laquelle la base organique comprend une amine aliphatique ou une amine aromatique présentant un caractère basique ou une autre base contenant de l'azote.

4. Microparticule selon l'une quelconque des revendications précédentes dans laquelle la base organique comprend un ou plusieurs composé parmi la N-méthylmorpholine, le N, N-diméthylaminoéthanol, la 4-diméthylaminopyridine, l'imidazol, le 1-méthylamidazole poly(chlorure de diallyldiméthylammonium) (PDAC), le chlorure de didécyldiméthylammonium (DDAC), la dodécyldipropylènetriamine (DDPT) et l'epsilon polylysine.

5. Microparticule selon l'une quelconque des revendications précédentes dans laquelle la base organique est déplacée avec une autre base réactive qui est alors mise en réaction afin de former une espèce réticulée.

6. Matériau macroporeux comprenant des microparticules selon l'une quelconque des revendications précédentes, les microparticules étant mises en contact afin de former des réticulations entre les microparticules et ainsi de former un corps tridimensionnel.

7. Utilisation d'une microparticule auto-assemblée selon l'une quelconque des revendications 1 à 5 ou d'un matériau macroporeux selon la revendication 6 en tant que macrostructure pour la culture cellulaire tridimensionnelle ou pour une utilisation dans des pansements à la place de collagène d'origine animale.

8. Utilisation selon la revendication 7 afin d'assurer la croissance de cellules osseuses.

9. Procédé de production d'une microparticule auto-assemblée ou d'un matériau macroporeux selon l'une quelconque des revendications 1 à 6 en milieux aqueux comprenant la mise en contact de l'acide de formule générale HOOC-(CH₂)ₙ-COOH dans lequel n est supérieur ou égal à 5 et inférieur à 40 avec une base organique dans un milieu aqueux dans lequel le rapport molaire des groupes acides sur les groupes basiques dans l'acide et la base est compris entre 0,6 et 1,4:1 et l'acide est insoluble dans le milieu aqueux et la base organique est soluble dans le milieu aqueux.

10. Composition antimicrobienne comprenant une microparticule auto-assemblée selon l'une quelconque des revendications 1 à 5.
